# EUROPÄISCHE PATENTSCHRIFT

(11) **EP 1 832 230 B1**
(45) Veröffentlichungstag und Bekanntmachung des Hinweises auf die Patenterteilung: **25.08.2010**
(21) Anmeldenummer: 06004601.8
(22) Anmeldetag: 07.03.2006
(51) Int. Cl.: A61B 5/103, A61B 5/11, G06F 19/00, G09B 23/30

(54) **Verfahren und Vorrichtung zur Erkennung und Lokalisierung eines Zusammenstosses von anatomischen Gelenkkomponenten**
Method and apparatus for detecting and localising an impingement between parts of an anatomical joint
Procédé et appareil de détection et localisation d'une empietement entre pièces d'une articulation anatomique

(43) Veröffentlichungstag der Anmeldung: 12.09.2007
(73) Patentinhaber: BrainLAB AG, 85622 Feldkirchen (DE)
(72) Erfinder: Götte, Hubert, 80333 München (DE); Immerz, Martin, 82166 Gräfelfing (DE)
(74) Vertreter: Schwabe - Sandmair - Marx

(56) Entgegenhaltungen:
- WO-A-02/02028
- US-A1- 2005 222 574
- US-A1- 2005 251 065
- US-B1- 6 708 142
- MOCTEZUMA J ET AL: "ROBOTIC SURGERY AND PLANNING FOR CORRECTIVE FEMUR OSTEOTOMY" PROCEEDINGS OF THE IEEE/RSJ/GI INTERNATIONAL CONFERENCE ON INTELLIGENTROBOTS AND SYSTEMS: ADVANCED ROBOTICS AND THE REAL WORLD. MUNICH, SEPT. 12 - 16, 1994, PROCEEDINGS OF THE INTERNATIONAL CONFERENCE ON INTELLIGENT ROBOTS AND SYSTEMS (IROS), NEW YOR, Bd. VOL. 2, 12. September 1994 (1994-09-12), Seiten 870-877, XP000514589 ISBN: 0-7803-1934-6

## Beschreibung

Die vorliegende Erfindung bezieht sich auf ein Verfahren und eine Vorrichtung zum Erkennen und bevorzugt auch zum Lokalisieren des Ortes eines Zusammenstoßes von Gelenkkomponenten eines Gelenkes, wie zum Beispiel eines Knie- oder Ellenbogengelenkes oder Hüftgelenkes.

Bei chirurgischen Eingriffen im Bereich eines anatomischen Gelenks, wie zum Beispiel bei und nach einer Implantation eines künstlichen Gelenks, sollte darauf geachtet werden, dass sich das Gelenk möglichst frei bewegen, also zum Beispiel schwenken und/oder drehen lässt, ohne dass eine zum Beispiel künstlich als Implantat eingesetzte Gelenkkomponente während einer normalen Gelenkbewegung an einer anderen Gelenkkomponente anstößt oder anliegt und bei einer Weiterbewegung des Gelenkes zum Beispiel verkantet, was zu einer nicht erwünschten übermäßigen Abnutzung oder Funktionsbeeinträchtigung des Gelenkes führen kann.

Bekannten chirurgischen Verfahren zur Folge sollten die Condylen vorsichtshalber immer anterior und posterior zurechtgeschnitten (trim) werden, wie zum Beispiel in "The Oxford Phase 3, Unicompartmental Knee, Manual of the Surgical Procedure" von Biomet Merck, "LCS Universal Kniesystem, OP-Technik" von DePuy oder "The M/G Unicompartmental Knee" von Zimmer beschrieben.

Um ein Zusammenstoßen (impingement) zu detektieren, ist ein als HIP ROM (Range of Motion) bezeichnetes Verfahren bekannt, welches mit der Vector-Vision-Hip-Software von BrainLAB implementiert werden kann. Dabei werden nach dem Pivotieren um den Mittelpunkt eines Hüftgelenks die Koordinaten des Pivotierungspunktes jeweils in Bezug auf das Becken und den Femur gespeichert, wobei an dem Becken und Femur jeweils Marker-Arrays angebracht sind, welche von einem Kamerasystem getrackt werden können. Während der Bewegung des Gelenks im normalen Funktionsbereich fallen die zwei Punkte in dem Koordinatensystem der Kamera zusammen, da das Hüftgelenk als Kugelgelenk angesehen werden kann. Tritt jedoch eine Verkantung und zum Beispiel ein Aushebeln einer Gelenkkomponente auf Grund einer Verkantung auf, so weichen diese zwei Punkte voneinander ab, was von einer Software erkannt werden kann, wobei jedoch keine Lokalisierung des Verkantungspunktes oder Auflagerpunktes der Gelenke möglich ist.

Aus der US 2005/0,113,720 A1 ist ein Verfahren zur Bestimmung des Drehmittelpunktes eines Knochens in einem Drehgelenk, wie zum Beispiel eines Femurs in dem Darmbein bekannt. Bezüglich der Messung der Positionen von Gelenkkomponenten oder Knochen wird auf die Lehre der US 2005/0,113,720 A1 verwiesen, deren diesbezügliche Lehre in diese Anmeldung aufgenommen wird.

Aus der DE 100 62 580 A1 ist das Bestimmen der mechanischen Achse eines Femurs bekannt, wobei auch die Lehre der DE 100 62 580 A1 bezüglich der Bestimmung der Lage einer Gelenkkomponente oder eines Knochens in diese Anmeldung aufgenommen wird.

Aus der WO 02/02028 ist ein Verfahren zur Detektion von zumindest zwei Körpern bekannt, umfassend im Wesentlichen die Schritte a) Erfassung von 3D-Bilddaten von den Körpern und darauf basierend Ermittlung eines Datensatzes von Punkten auf der Körperoberfläche, b) Anfügen eines Referenzelements mit zumindest drei Markern an jeden der Körper und Messen der 3D-Koordinaten der Marker mit Bezug auf ein relatives Koordinatensystem mittels einer Positions-Messvorrichtung während der Bewegung der Körper; c) Berechnung mittels eines Computers ob und wo zwei Punkte, die jeweils auf den Oberflächen der Körper am nächsten zueinander liegen, in dem relative Koordinatensystem zusammentreffen.

Aus der US 6,705,142 B1 ist ein Algorithmus zur Modellierung der Zwischenwirkung von zwei kollidierenden steifen Körpern bekannt, welcher die Suche einer optimalen Position und einer Stabilisierung der steifen Körper gestattet, sofern die Körper Kontakt zueinander haben aufgrund einer Kraft, die sie gegeneinander stößt.

Allgemein können die Positionen von Gelenkkomponenten oder Knochen zum Beispiel durch an den Knochen angebrachte Marker-Arrays oder Referenzsterne oder mit den Knochen unmittelbar oder mittelbar verbundene Elemente, wie zum Beispiel Markern, welche lokalisiert werden können, ermittelt werden.

Es ist eine Aufgabe der vorliegenden Erfindung ein Zusammenstoßen (impingement) von Komponenten eines anatomischen Gelenkes zu detektieren.

Diese Aufgabe wird durch ein Verfahren und eine Vorrichtung mit den Merkmalen der unabhängigen Patentansprüche gelöst. Vorteilhafte Ausführungsformen ergeben sich aus den abhängigen Patentansprüchen.

Bei einem erfindungsgemäßen Verfahren zum Erkennen eines Zusammenstoßes (impingement) von Gelenkkomponenten eines anatomischen Gelenks, wie zum Beispiel eines Hüft-, Schulter-, Ellenbogengelenks oder im Fall des Kniegelenks beim Zusammenstoßen des Femurs mit der Tibia mit oder ohne eingesetzten künstlichen Gelenken oder Gelenkteilen, wird die räumliche Lage jeder Gelenkkomponente detektiert, wobei die Gelenkkomponenten relativ zueinander bewegt werden, um so aus der sich verändernden räumlichen Relativlage der Gelenkkomponenten den Ort der momentanen Drehachse zu berechnen, welche zum Beispiel bei einem Kniegelenk durch den Femur verlaufen kann.

Wenn die Gelenkkomponenten während einer kontinuierlichen Bewegung an einer Stelle zusammenstoßen, anstatt das für das Kniegelenk typische Rollgleiten auszuführen, wird ein Erwartungsbereich für die (Weiter-) Bewegung der momentanen Drehachse oder eines momentanen Drehmittelpunktes verlassen, zum Beispiel wird sich die momentane Drehachse des Kniegelenks aus ihrem normalen oder definierten Arbeitsbereich heraus bewegen oder beispielsweise bei einem harten Zusammenstoß ihre Lage sprunghaft verändern. In einem anderen Fall kann durch das Blockieren der weiteren Bewegung die Drehachse im Arbeitsbereich bleiben, sich aber nicht mehr weiterbewegen und so den normalen oder physiologischen Arbeitsbereich in ihrer Bewegung nicht mehr ganz ausfüllen. In bestimmten Fällen wird die neu ermittelte Drehachse durch den Ort verlaufen, an dem die Gelenkkomponenten zusammenstoßen, so dass sich die Gelenkskomponenten nicht mehr relativ zueinander wie gewünscht durch eine Gelenkverbindung definiert bewegen, sondern an der Zusammenstoß- oder Kipplinie kippen, was zu einer verstärkten Abnutzung des Gelenks und zu einem Herausbewegen einer Gelenkkomponente aus dem zum Funktionieren als Gelenk erforderlichen Sitz führen kann. Das Gelenk kann demzufolge verrenkt oder ausgekugelt werden, was auch als Luxation bezeichnet wird. Bei einem Kippen werden sich die momentanen Drehachsen in einer gewissen Umgebung nahe um die Kipplinie herum konzentrieren, was erfindungsgemäß detektiert werden kann.

Erfindungsgemäß wird ein Erwartungsbereich für die Bewegung oder Weiterbewegung einer momentanen Drehachse oder eines momentanen Drehmittelpunktes bestimmt oder vorausberechnet, um die wahrscheinliche oder vermutete räumliche Bewegung der momentanen Drehachse oder des momentanen Drehmittelpunktes vorzugeben, zum Beispiel durch ein definiertes Volumen, innerhalb dessen sich der momentane Drehmittelpunkt oder die momentane Drehachse bewegt. Wird dieser Erwartungsbereich verlassen, so kann erfindungsgemäß festgestellt werden, dass die Gelenkkomponenten zusammengestoßen sind. Ein solcher räumlich definierter Erwartungsbereich kann auch als globaler Erwartungsbereich bezeichnet werden, welcher einen möglichen zeitlichen Bewegungsverlauf innerhalb gewisser Grenzen, also zum Beispiel innerhalb eines vorgegebenen oder definierten Volumens, für die momentane Drehachse oder den momentanen Drehmittelpunkt vorgibt. Ebenso kann erfindungsgemäß alternativ oder ergänzend auch ein lokaler Erwartungsbereich vorgegeben werden, welcher ausgehend von einer aktuellen Position der Drehachse oder des Drehmittelpunktes vorgibt, in welchem Abstand die nächste ermittelte momentane Drehachse oder der nächste ermittelte momentane Drehmittelpunkt liegen kann, so dass zum Beispiel beim Verlassen des lokalen Erwartungsbereiches festgestellt werden kann, dass ein Zusammenstoß von Gelenkkomponenten vorliegt. Dabei kann es auch vorkommen, dass zwar der lokale Erwartungsbereich verlassen wird, jedoch ein neu ermittelter Drehmittelpunkt oder eine neu ermittelte Drehachse noch im globalen Erwartungsbereich liegt. Unabhängig davon, welcher Erwartungsbereich verwendet wird, kann erfindungsgemäß beim Verlassen des Erwartungsbereiches festgestellt werden, dass ein Zusammenstoß der Gelenkkomponenten vorliegt. Mittels einer Bewegungsanalyse kann zum Beispiel ein solcher lokaler oder globaler Erwartungsbereich vorgegeben werden, welcher eine Erwartungshaltung bezüglich des Ablaufes einer Bewegung zweier Gelenkkomponenten relativ zueinander darstellt und welcher definieren kann, ob die Bewegung gleichmäßig, glatt oder stetig abläuft oder ob unerwartete Ereignisse, also zum Beispiel Sprünge, vorliegen, so dass auf einen Zusammenstoß von Gelenkkomponenten geschlossen werden kann. Die nachfolgenden Ausführungen, welche sich zum Beispiel auf definierte Volumen oder definierte Flächen beziehen, geben über diese definierten Volumen oder Flächen globale und/oder lokale Erwartungsbereiche vor.

Ein Erwartungsbereich kann erfindungsgemäß auch so eingegrenzt sein, dass der momentane Drehmittelpunkt oder die momentane Drehachse innerhalb oder außerhalb eines vordefinierten Plausibilitätsbereiches liegt, also zum Beispiel innerhalb einer Gelenkstruktur, wie zum Beispiel dem Femur, um zum Beispiel auszuschließen, dass von einem außerhalb des Femurs liegenden Drehmittelpunkt ausgegangen wird.

Erfindungsgemäß kann ein Zusammenstoß von Gelenkkomponenten eines Gelenks dadurch erkannt werden, dass die räumliche Lage jeder Gelenkkomponente während einer Relativbewegung der Gelenkkomponenten detektiert wird, der momentane Drehmittelpunkt oder die momentane Drehachse aus den detektierten räumlichen Lagen der Gelenkkomponenten berechnet wird und festgestellt wird, wie sich bei einer Bewegung der einen Gelenkkomponente relativ zur anderen Gelenkkomponente der berechnete Drehmittelpunkt entlang einer Kurve bewegt, die durch Interpolation über alle Drehmittelpunkte in der Reihenfolge ihres Durchlaufens berechnet wird, oder wie sich die berechnete Drehachse auf einer gekrümmten Fläche bewegt, die durch Interpolation über alle Drehachsen in der Reihenfolge ihres Durchlaufens berechnet wird, wobei im Falle des Vorliegens von Kurvenstücken mit einer Krümmung über einem vorgegebenen Grenzwert oder von Kurvenstücken mit einem Abstand der benachbarten Drehmittelpunkte über einem vorgegebenen Grenzwert oder von Flächenstücken mit einer Krümmung über einem vorgegebenen Grenzwert oder von Flächenstücken mit einem großen Abstand der benachbarten Achsen über einem vorgegebenen Grenzwert festgestellt wird, dass ein Zusammenstoß der Komponenten vorliegt. Bei der Interpolation kann eine Ausreißerbehandlung vorgenommen werden. Die Ausdehnung der Fläche, die aus den ins Unendliche reichenden Drehachsen berechnet wird, kann auch durch ein vorgegebenes Volumen mit endlicher Ausdehnung begrenzt werden.

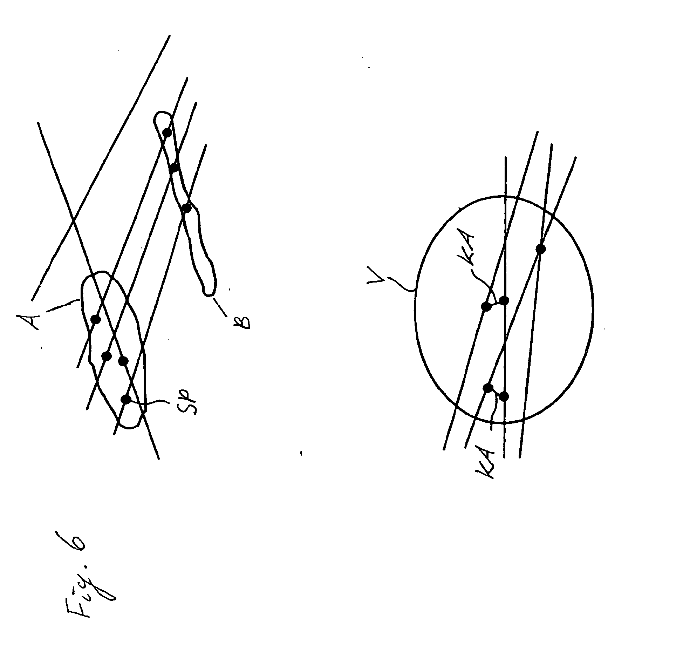

Die Erfindung erkennt das wahrscheinliche Vorliegen eines Zusammenstoßes durch die Überwachung der Position der momentanen Drehachse. Bei einem Kniegelenk werden mehrere oder zumindest eines der folgenden Kriterien überwacht oder detektiert:
- ob die Drehachse eine oder mehrere definierte begrenzte Flächen schneidet (siehe Figur 6), wobei die definierte Fläche auch die Hülle eines definierten Volumens sein kann.
- ob sich die Drehachse in einem zum Beispiel vorher definierten Volumen oder über eine definierte Fläche bewegt,
- ob die jeweilige Veränderung der Lage und der Orientierung der neu ermittelten momentanen Drehachse zu einer beliebigen vorherigen bestimmten Drehachse innerhalb vorgegebener Grenzen liegt,
- ob sich die Durchstoßpunkte der Drehachsen (also die "Drehzentren") durch die Sagittalebene zum Beispiel in der Mitte des Knies innerhalb eines definierten Bereiches oder entlang einer definierten Kurve (zum Beispiel der sog. Gangpolkurve) bzw. in ihrer Nähe bewegen,
- ob die ermittelte oder momentane Drehachse oder das Drehzentrum einen vorgebenden Bereich ganz oder nur teilweise überstreicht, oder
- ob sich die Drehachsen in einem definierten Mindestabstand zu den Oberflächen der Gelenkkomponenten, also zum Beispiel von Tibia oder Femur oder der eingesetzten Gelenkkomponenten an Tibia oder Femur befinden.

Werden eines oder mehrere dieser Kriterien nicht erfüllt, liegt wahrscheinlich ein Zusammenstoß vor. Der mögliche Ort des Zusammenstoßes kann ermittelt werden, indem der Drehachsenverlauf innerhalb eines definierten oder abgegrenzten Volumens untersucht wird. Bei der Bestimmung des wahrscheinlichsten Ortes kann zum Beispiel ein iteratives Verfahren angewendet werden, bei dem im Suchvolumen zunächst ein Teilvolumen definierter Ausdehnung bestimmt wird. In dem Teilvolumen wird die Häufung von Kreuzungspunkten und kürzesten Abständen zwischen Drehachsen (siehe Figur 6) bestimmt. Durch Wandern des Teilvolumens wird das gesamte Suchvolumen überstrichen. Das Teilvolumen mit der größten Häufung kann dann als neues Suchvolumen herangezogen und wieder einer genaueren Suche unterzogen werden. Liegt ein gemeinsamer Schnittpunkt oder eine Konzentration der Drehachsen oder ihrer Schnittpunkte in diesem Volumen vor, so kann sich der Ort des Zusammenstoßes in diesem Volumen befinden. Es kann dabei überprüft werden, ob der ermittelte Ort innerhalb oder außerhalb eines vordefinierten Plausibilitätsbereichs liegt, um falsche Orte auszuschließen. So werden sich beispielsweise bei einem Blockieren der Rollbewegung die Drehachsen bei einem reinen Durchgleiten des Femurs zwar in einem engen Volumen innerhalb des Kniegelenks konzentrieren, aber dieser Ort wird im Inneren des distalen Femurs liegen und nicht der Ort des Zusammenstoßens bzw. der Blockade sein können, der am Rand der Knochen-, oder Gelenkstruktur, in diesem Falle des Femurs liegen muss. Die falsche Ermittlung wird dadurch ausgeschlossen, dass das Innere des Femurs als vordefinierter Bereich beim Ausschluss berücksichtigt wird. Der übliche Bewegungsbereich der Drehachse des Kniegelenks kann zum Beispiel der Untersuchung von H.H. Wetz und H.A.C. Jacob, "Use of spatial motion of the femorotibial joint for the alignment of knee braces" in Orthopädie, 2001, 30:196-207, Springer-Verlag 2001 entnommen werden.

Im Beispiel eines Hüftgelenks wäre die normale Funktion gegeben, wenn sich die ermittelten momentanen Drehachsen des Femurs relativ zum Pelvis immer in einem Punkt schneiden oder in einem bestimmten Bereich oder Volumen konzentrieren. Wenn die Drehachse sich nicht mehr in diesem Punkt mit den anderen schneidet oder durch ein vorgegebenes Volumen verläuft, verlässt das Gelenk den normalen Arbeitsbereich und es kommt zu einem Zusammenstoß der Gelenkskomponenten an der entstehenden Kipplinie, welche erfindungsgemäß detektiert werden kann.

Analog können bei einem Hüftgelenk mehrere oder mindestens eines der folgenden Kriterien überwacht werden:
- ob sich die Drehachsen immer in einem gemeinsamen Schnittpunkt bzw. einem definierten Volumen um diesen schneiden bzw. durch das definierte Volumen verlaufen,
- ob sich die Drehachsen in einem definierten Mindestabstand zu den Oberflächen von Femur oder Tibia befinden.

Werden eines oder mehrere dieser Kriterien verletzt, liegt wahrscheinlich ein Zusammenstoß vor. Der mögliche Ort des Zusammenstoßes wird ermittelt, indem der Drehachsenverlauf innerhalb eines definierten Volumens untersucht wird. Bei der Bestimmung des wahrscheinlichsten Ortes kann zum Beispiel ein iteratives Verfahren angewendet werden, bei dem im Suchvolumen zunächst ein Teilvolumen definierter Ausdehnung bestimmt wird. In dem Teilvolumen wird die Häufung von Kreuzungspunkten und kürzesten Abständen zwischen Drehachsen (siehe Figur 6) bestimmt. Durch Wandern des Teilvolumens wird das gesamte Suchvolumen überstrichen. Das Teilvolumen mit der größten Häufung kann dann als neues Suchvolumen herangezogen und wieder einer genaueren Suche unterzogen werden. Liegt ein gemeinsamer Schnittpunkt oder eine Konzentration der Drehachsen oder ihrer Schnittpunkte vor, so kann sich der Ort des Zusammenstoßes in dieser Umgebung befinden. Es wird wie beim Beispiel des Kniegelenks überprüft, ob der ermittelte Ort innerhalb oder außerhalb eines vordefinierten Bereichs liegt, um falsche Orte auszuschließen.

Sofern in dieser Anmeldung von einer definierten oder vorgegebenen Fläche oder einem Bereich gesprochen wird, sollen darunter auch Flächen in Form eines Kreises, einer Ellipse, eines Rechtecks oder eines anderen abgegrenzten Umrisses verstanden werden, wobei die Fläche zum Beispiel durch einen definierten Abstand von einem Punkt, wie zum Beispiel einem Drehpunkt (Kreis) oder einer Linie oder Kurve, zum Beispiel der Gangpolkurve oder einem Knochenumriss, definiert sein kann. Der Abstand kann zum Beispiel im Bereich von 0 bis 10 cm liegen und zum Beispiel 0 bis 5 mm, 10 mm, 15 mm oder 20 mm betragen. Die Fläche kann auch gekrümmt und/oder aus Teilstücken zusammengesetzt sein.

Sofern in dieser Anmeldung von einem definierten oder vorgegebenen Volumen oder räumlichen Bereich gesprochen wird, sollen darunter Volumen zum Beispiel in Form einer Kugel, eines Quaders, eines Zylinders oder eines anderen abgegrenzten geometrischen Körpers verstanden werden, wobei das Volumen zum Beispiel durch einen definierten Abstand von einem Punkt, wie zum Beispiel Drehpunkt, (Kugel) oder von einer Achse, wie zum Beispiel einer Drehachse, (Zylinder) oder von einer Kurve oder Linie, zum Beispiel der Gangpolkurve oder von einer Gelenkkomponente oder einem Knochen definiert sein kann. Der definierte Abstand kann zum Beispiel im Bereich von 0 bis 10 cm liegen und zum Beispiel 0 bis 5 mm, 10 mm, 15 mm oder 20 mm betragen. Das Volumen kann auch aus mehreren Teilvolumina zusammengesetzt sein.

Da anatomische Gelenke, wie zum Beispiel das Kniegelenk, keine idealen Kugelgelenke sind, haben anatomische Gelenke meist auch keine feststehende Drehachse. Vielmehr bewegt sich die momentane Drehachse mit der Bewegung des Gelenks bzw. der Gelenkkomponenten, so dass die momentane Drehachse durch eine so genannte Bewegungsanalyse lokalisiert werden muss, wobei die Positionen der Gelenkkomponenten erfasst werden und aus der erfassten Relativbewegung der Gelenkkomponenten die momentane Drehachse berechnet wird.

Rodriguez hat ein rechnerisches Verfahren beschrieben, um aus zwei räumlichen Positionen eines Körpers die zugrunde liegende Bewegung aus Rotation und Translation zu bestimmen; siehe Bisshopp, K. E.: "Rodriguez formula and the screw matrix" in: Journal of engineering for industry, Transactions of the ASME (1969), Seiten 179-185. Panjabi hat diese Methode in die Biomechanik eingeführt; siehe Panjabi, M.; White, A.A.: "A mathematical approach for three-dimensional analysis of the mechanics of the spine" in: Journal of Biomechanics 4 (1971), Nr. 3, Seiten 203-211. Woltring geht von einer Transformationsmatrix zur Beschreibung der Orientierung und Position der räumlichen Positionen aus; siehe Woltring, H.J.: "Representation and calculation of 3-D joint movement" in: Human Movement Science 10 (1991), Seiten 603-616; Woltring, H.J.: "3-D attitude representation of human joints: a standardization proposal" in: Journal of Biomechanics 27 (1994), Nr. 12, Seiten 1399-1414; Woltring, H.J.; Huiskes, R.; Lange, A. de; Veldpaus, F.E.: "Finite centroid and helical axis estimation from noisy landmark measurements in the study of human joint kinematics" in Journal of Biomechanics 18 (1985), Nr. 5, Seiten 379-389.

Vor dem Bewegen einer Gelenkkomponente über den Bewegungsbereich (Range Of Motion) des Gelenkes wird bevorzugt ein Referenz-Array, wie zum Beispiel ein mit passiven Markern versehener Referenzstern, an den Gelenkkomponenten, wie zum Beispiel dem Femur und der Tibia angebracht. Werden diese Referenz-Arrays mit einer Tracking-Vorrichtung, wie zum Beispiel einem Kamerasystem, getrackt, können die momentanen Drehachsen aus den getrackten Positionsdaten berechnet werden.

Menschik, A.: "Biometrie- das Konstruktionsprinzip des Kniegelenks, des Hüftgelenks, der Beinlänge und der Körpergröße", Springer, 1987 und andere haben das Kniegelenk als überschlagene Viergelenkskette beschrieben. Wenn das Femur fixiert wird (Rastsystem), kann die Tibia in der Sagittalebene bewegt werden (Gangsystem). Die Menge der durch die Tibia erzeugten momentanen Drehzentren bildet die Rastpolkurve. Wird die Tibia fixiert und das Femur bewegt, stellen die momentanen Drehzentren die Gangpolkurve dar.

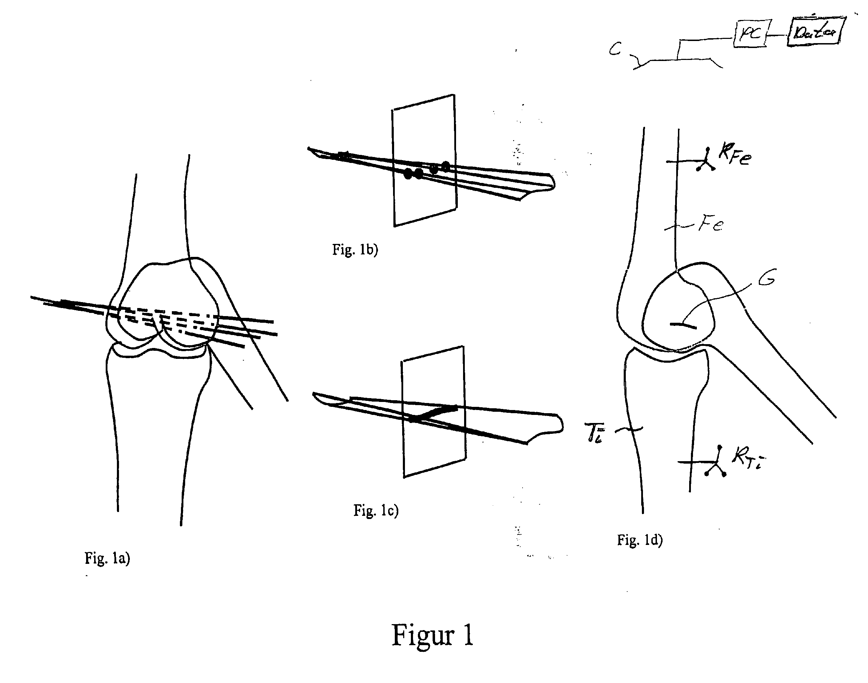

Betrachtet man zum Beispiel ein Kniegelenk in der sagittalen zweidimensionalen Projektion (von seitlich), so durchstoßen die durch den Femur verlaufenden momentanen Drehachsen (siehe Figur 1 a) als Drehzentren die sagittale Ebene durch die Kniegelenksmitte (siehe Figuren 1b-d).

Wird die Position des Femurs in Bezug auf die Tibia getrackt, werden die ermittelten Drehzentren des Femurs demnach auf oder in der Nähe der Gangpolkurve liegen.

Wenn für die Überwachung der Drehachsen ein vordefinierter Bereich benutzt werden soll und dieser Bereich beispielsweise die Gangpolkurve ist, kann sie näherungsweise zum einen durch Ermittlung der Drehzentren in einem Bewegungsbereich definiert werden, in dem sicher keine Kollision vorliegt. Nach der Modellvorstellung von Menschik entspricht auch der Schnittpunkt der Kanten, die das hintere und das vordere Kreuzband in der sagittalen Projektion bilden, dem momentanen Drehzentrum. Um den Verlauf der Gangpolkurve mit diesem Prinzip zu definieren, wird der Kreuzungspunkt der Verbindungslinien zwischen den femoralen und den tibialen Ansatzpunkten gebildet und über den mittleren Bereich der Gelenkbewegung bestimmt, der sicher nicht zu einer Kollision der Gelenkkomponenten führt.

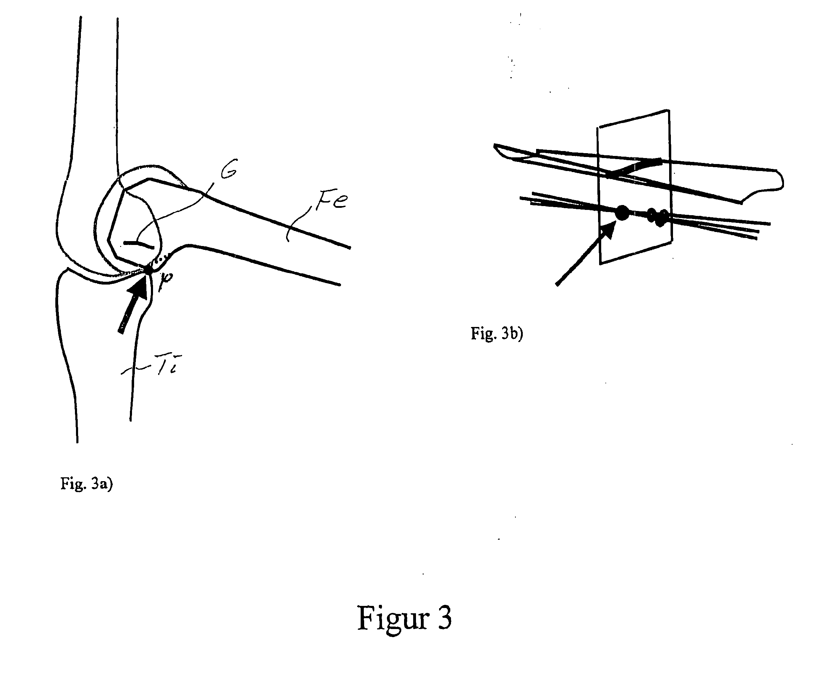

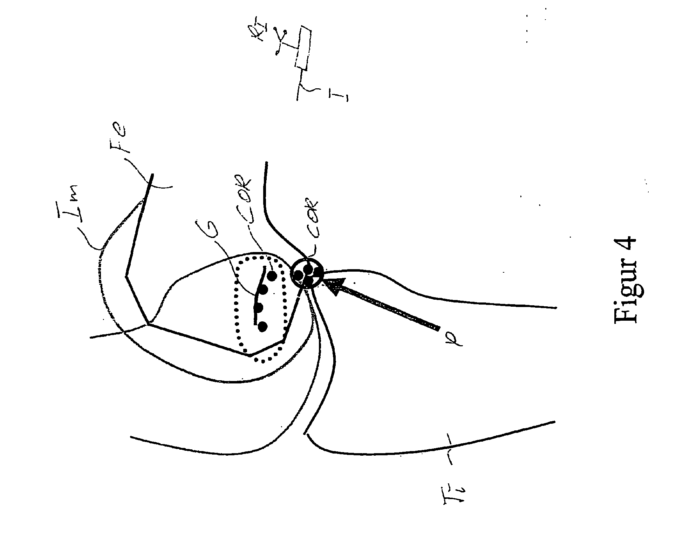

Wenn die berechneten momentanen räumlich verlaufenden Drehachsen der Bewegung oder Abwinkelung des Kniegelenks beginnen, von dem vordefinierten Bereich (der zum Beispiel in der sagittalen Projektion der Drehzentren die Gangpolkurve sein kann) zum Beispiel um mehr als einen vorgegebenen Abstand von zum Beispiel 1 bis 50 mm abzuweichen und sich dem posterioren Teil des Tibiaplateaus annähern und sich an einem bestimmten Punkt oder abgegrenzten oder vorgegebenen Bereich sammeln, während die Abwinkelung des Femurs erhöht wird, ist es sehr wahrscheinlich, dass ein Zusammenstoßen bei diesem bestimmten Punkt auftritt, wie durch den Punkt oder Bereich P in Figur 3a oder Figur 3b oder Figur 4 gezeigt wird, so dass erfindungsgemäß ein Zusammenstoß erkannt und auch an der Stelle des Punktes P lokalisiert werden kann, wenn sich die momentane Drehachse nicht mehr in dem vordefinierten Bereich bewegt, sondern zu einem Punkt oder Bereich P springt, welcher zum Beispiel am Rande eines für das Funktionieren des Gelenkes vorgesehenen funktionalen Gelenkbereiches einer oder beider Gelenkkomponenten liegen kann. Diese Lokalisierung kann auch intraoperativ erfolgen. Die Lokalisierung kann sowohl in der zweidimensionalen sagittalen Projektion der Drehzentren erfolgen und dabei einen Punkt ergeben, durch den die Kipplinie verläuft und sie kann räumlich erfolgen, indem die Konzentration der Drehachsen in einem engen räumlichen Bereich identifiziert wird und der Punkt des Zusammenstoßens oder der räumliche Verlauf der Kipplinie in diesem Bereich lokalisiert wird.

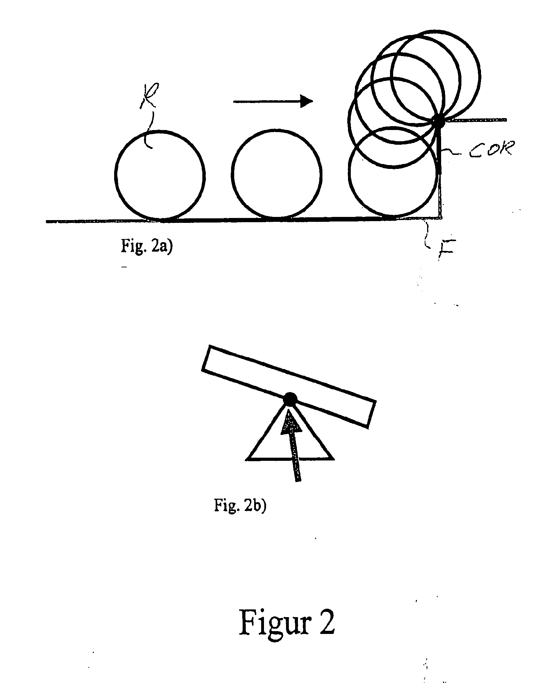

Zur allgemeinen Erläuterung des Funktionsprinzips der vorliegenden Erfindungen wird auf die Figuren 2a und 2b verwiesen, wobei in Figur 2a die Bewegung des momentanen Drehmittelpunktes COR eines auf einer Auflagefläche F abrollenden Rades R in Pfeilrichtung gezeigt ist und erkannt werden kann, dass sich der momentane Drehmittelpunkt COR entlang einer Gerade auf Höhe des Bodens bewegt. Kommt das Rad R zum Beispiel an eine Begrenzung auf der Fläche F, so dass das Rad R nicht mehr auf der Fläche F abrollen kann, so blockiert das Rad und begrenzt damit den weiteren Verlauf des Drehmittelpunktes auf dem Boden (siehe Figur 2a). Wenn das Rad nach oben weiter bewegt wird, kippt es über den Auflagepunkt, wobei der neue momentane Drehmittelpunkt nicht mehr über den Boden wandert, sondern sich im Bereich des Auflagepunktes konzentriert, so dass sich das nicht mehr abrollende Rad R wie eine in Figur 2b gezeigte Wippe verhält, deren momentaner Drehmittelpunkt COR immer auf dem durch den Pfeil hervorgehobenen Auflagepunkt liegt.

Diese Erkenntnis kann bei der Bestimmung des Bewegungsbereiches eines anatomischen Gelenks ausgenutzt werden, wie schematisch in den Figuren 1 a und 1b dargestellt ist. Ähnlich wie bei einer Wippe wird sich das erfindungsgemäß gemessene oder bestimmte momentane Drehzentrum während eines Zusammenstoßes und Verkippens der Gelenkkomponenten nur an dem Auflagepunkt P befinden oder sich in einem geringen Abstand von zum Beispiel 0 bis 10 mm dazu bewegen, wie in Figur 3a oder 4 gezeigt, wobei dieser Übergang des momentanen Drehzentrums in den Kollisionsbereich durch das Erfassen der Momentanpositionen der Gelenkkomponenten festgestellt werden kann und wobei vorteilhaft ein vordefinierter Arbeitsbereich, gegeben zum Beispiel durch die Gangpolkurve oder einen anderen sinnvollen Verlauf, zur Bestimmung, ob sich das Gelenk noch im normalen Arbeitsbereich befindet, herangezogen werden kann, so dass bei einem Abweichen des momentanen Drehzentrums von dem vorgegebenen Bereich um zum Beispiel mehr als ein vorgegebener Abstand von zum Beispiel 0 bis 20 mm ein Zusammenstoßen detektiert werden kann und durch die Untersuchung des weiteren Verlaufs des Drehzentrums auf die Konzentration um einen Punkt P lokalisiert werden kann.

Allgemein können voneinander unabhängige Messverfahren verwendet werden, welche auch beliebig kombiniert werden können, wobei nachfolgend acht Kriterien oder Ausführungsbeispiele angegeben sind:

***Erstens*** kann die Abweichung des momentanen Drehzentrums (also die Durchstoßpunkte der Drehachsen in der Projektionsebene) von einem vorgegebenen Verlauf (zum Beispiel der Gangpolkurve) oder Bereich beobachtet werden, indem zum Beispiel ermittelt wird, ob das momentane Drehzentrum in der sagittalen Projektionsebene (oder auch in einer anderen beliebig orientierten geeigneten Projektionsebene) in einem vorgegebenen Abstand von zum Beispiel 5 oder 10 mm um den vorgebenden Verlauf oder Bereich herum liegt, wie durch die gepunktete Linie in Figur 4 gezeigt.

***Zweitens*** kann untersucht werden, ob sich die räumlich verlaufende Drehachse mindestens mit einer vorgegebenen Strecke in einem definierten Volumen, wie zum Beispiel einem Quader, Zylinder, Kugel befindet, oder immer eine definierte Fläche oder mehrere definierte Flächen schneidet.

***Drittens*** kann ermittelt werden, ob die jeweilige Veränderung der Lage und der Orientierung der ermittelten Drehachse zu einer beliebigen vorherigen bestimmten Drehachse innerhalb oder außerhalb vorgegebener Grenzen liegt, zum Beispiel um das Springen der Achse festzustellen.

Die Lageänderung kann zum Beispiel dadurch überprüft werden, dass ein begrenztes Volumen definiert wird, das von der einen Achse durchlaufen wird und in dem sich auch Punkte aller anderen zu überprüfenden Achsen befinden müssen. Jede Achse kann der Prüfung zugrunde gelegt werden und entsprechend kann das Volumen für jede Achse neu definiert werden. Die Orientierung kann zum Beispiel dadurch überprüft werden, dass zwei berandete Flächen definiert werden, die von der einen Achse geschnitten werden und die beide auch von jeder der anderen Achsen geschnitten werden müssen. Jede Achse kann der Prüfung zugrunde gelegt werden und entsprechend können die Flächen für jede Achse neu definiert werden.

***Viertens*** kann ermittelt werden, ob sich die Drehachsen in einem definierten Mindestabstand zu den Oberflächen der Gelenkkomponenten, also zum Beispiel von Tibia oder Femur, oder den eingesetzten Gelenkkomponenten an Tibia oder Femur befinden.

***Fünftens*** kann ermittelt werden, ob die Drehachse oder das Drehzentrum einen vorgebenden Bereich ganz oder nur teilweise (siehe Figur 2a) überstreicht, um zum Beispiel ein Blockieren innerhalb des Arbeitsbereiches festzustellen und von einem Kippen um einen Kollisionspunkt unterscheiden zu können.

***Sechstens*** kann der mögliche Ort des Zusammenstoßes ermittelt werden, indem iterativ der Drehachsenverlauf im Bereich des Zusammenstoßes auf eine Konzentration der Drehachsen innerhalb eines abgegrenzten Volumens, wie oben beschrieben, untersucht wird. Liegt eine Vielzahl gemeinsamer Schnittpunkte oder eine Konzentration der Drehachsen oder Punkte der Achsen in diesem Volumen vor bzw. wird ein Teilvolumen mit einer Häufung von Kreuzungspunkten gefunden, so kann sich der Ort des Zusammenstoßes in diesem Volumen befinden; siehe Figur 3b.

***Siebtens*** kann in der Projektion (zum Beispiel Sagittalprojektion) die Konzentration der Drehzentren in einem engen Bereich detektiert werden, um einen Verlauf der Kipplinie durch diesen Bereich zu lokalisieren.

***Achtens*** kann überprüft werden, ob der ermittelte Ort oder Punkt innerhalb oder außerhalb eines vordefinierten Plausibilitätsbereichs, insbesondere innerhalb einer Gelenkstruktur, wie zum Beispiel dem Femur, liegt, um falsche Orte auszuschließen. Der Plausibilitätsbereich kann eine definierte Fläche oder ein Volumen sein.

Eine allgemeine Bewegung eines starren Körpers setzt sich aus einer Rotation und einer Längsverschiebung, also einer Translation, zusammen. Die Drehachse kann sich dabei jedoch von Augenblick zu Augenblick im Raum verschieben, so dass sich die Richtung und der Ort der momentanen Drehachse ändern können. Betrachtet man zum Beispiel das rollende Rad eines Wagens, so stellt man fest, dass die momentane Drehachse des Rades mit der Geschwindigkeit des Wagens wandert und durch den Punkt geht, an dem das Rad den Boden berührt.

Sucht man den aktuellen räumlichen Ort der Drehachse, kann die sog. "Helical Axis"- oder "Schraubachsen"- Methode angewendet werden.

Basierend auf dem Lehrsatz von Mozzi-Chasles', der besagt, dass jede Bewegung starrer Körper in eine ebene Rotation um eine geeignete Achse, gefolgt von einer Translation entlang derselben Achse, zerlegt werden kann, hat Rodrigues a.a.O. ein rechnerisches Verfahren beschrieben, um aus zwei räumlichen Positionen eines Körpers die zugrunde liegende Bewegung aus Rotation und Translation zu bestimmen. Panjabi a.a.O. hat die Methode in die Biomechanik eingeführt. Woltring a.a.O. geht von einer Transformationsmatrix aus. Die bekannte Rollbewegung eines Rades ist ein Sonderfall, bei dem die Translation entlang der momentanen Drehachse verschwindet.

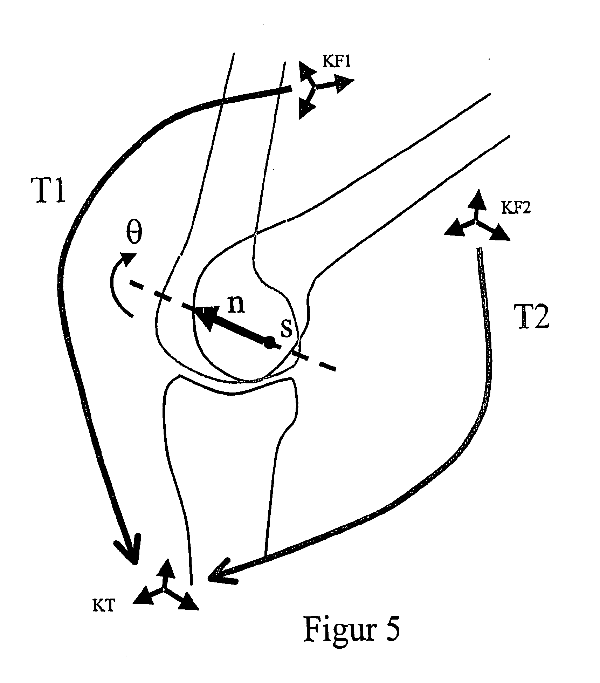

Die Berechnung der momentanen Drehachse kann, wie unter Bezugnahme auf Figur 5 erläutert, durchgeführt werden. Aus den Transformationsmatrizen T1 und T2, welche eine stückweise Positionsänderung des mit dem Femur verbundenen Referenzsternes während einer Abwinkelung (Flexion) des Gelenkes gegenüber der Tibia beschreiben, kann die finite Rotationsachse (als Schraubachse oder *helical axis)* mit dem Richtungsvektor n und einem Punkt S in Koordinaten des Tibiasystems und der Betrag der Drehung θ erhalten werden. Zu Details der möglichst störunanfälligen Berechnung siehe Woltring a.a.O.. Indem die gesamte Bewegung des Gelenks in eine Abfolge von solchen kleinen Positionsänderungen zerlegt wird, kann der stückweise Verlauf der Drehachse bestimmt werden. Das Verfahren kann jedes Mal durchgeführt werden, wenn eine Bewegung vollzogen wird.

Somit kann erfindungsgemäß das Zusammenstoßen von Komponenten eines anatomischen Gelenkes durch Untersuchung der genannten Kriterien detektiert werden. Der Ort des Zusammentreffens muss auf der momentanen Drehachse liegen. Falls festgestellt wird, dass kein Zusammenstoß während der Bewegung des Gelenks über den gewünschten Bewegungsbereich auftritt, ist es nicht mehr erforderlich, das sonst übliche Zurechtschneiden von Gelenkkomponenten eines anatomischen Gelenks vorzunehmen, wodurch einerseits gesundes Körpergewebe erhalten bleibt und andererseits Zeit gespart wird. Wenn ermittelt wird, wo ein Zusammentreffen der Gelenkkomponenten stattfindet, kann ein Chirurg die Gelenkkomponenten entsprechend verändern und zum Beispiel Punkte oder Flächen, welche für das unerwünschte Zusammenstoßen der Gelenkkomponenten verantwortlich sind, herausschneiden (trimming) und so das Zusammentreffen der Gelenkkomponenten und damit die Einschränkung des Bewegungsbereiches des anatomischen Gelenks effektiver beseitigen.

Vorzugsweise kann auch ein chirurgisches Instrument, welches zum Beispiel zum Schneiden einer Gelenkkomponente, wie zum Beispiel den Condylen, bewegt wird, zu der erfindungsgemäß ermittelten Stelle des Zusammenstoßes navigiert werden. Dies ist insbesondere bei minimal invasiven Verfahren vorteilhaft, bei welchen im Allgemeinen keine freie Sicht auf das Gelenk gegeben ist.

Erfindungsgemäß ist es somit möglich, den maximalen Bewegungsbereich zuverlässig zu ermitteln, bevor weitere physiologische Begrenzungen oder Beschädigungen, zum Beispiel der Kreuzbänder, auftreten, was bei konventionellen Verfahren möglich sein kann.

Weiterhin bezieht sich die Erfindung auf ein Computerprogramm, welches, wenn es auf einem Computer läuft oder in einen Computer geladen ist, einen oder mehrere der oben beschriebenen Verfahrensschritte ausführt, sowie auf ein Programmspeichermedium oder Computerprogrammprodukt mit einem solchen Computerprogramm.

Gemäß einem weiteren Aspekt bezieht sich die Erfindung auf eine Vorrichtung zur Durchführung eines wie oben beschriebenen Verfahrens.

Eine erfindungsgemäße Vorrichtung weist mindestens eine Erfassungseinheit auf, welche die räumliche Position von Referenz-Arrays, welche an Gelenkkomponenten angebracht sind, erfassen kann. Diese Erfassungseinheit, wie zum Beispiel eine Infrarot-Kamera, ist mit einem Computer verbunden, welcher aus den erfassten Positionen der mit den Gelenkkomponenten verbundenen Referenz-Arrays eine momentane Drehachse oder einen momentanen Drehmittelpunkt oder ein Drehzentrum der relativ zueinander bewegten und durch ein Gelenk verbundenen Gelenkkomponenten berechnen kann, wobei der Computer weiterhin feststellen kann, ob sich die momentanen Drehpunkte entlang einer zu interpolierenden Kurve mit definiertem maximalen Krümmungsradius bewegen und einen gegebenen Mindestabstand zu der Kurve oder voneinander überschreiten bzw. nicht überschreiten, oder ob die momentanen Drehachsen eine zu interpolierende Fläche im Raum mit definierter maximaler Krümmung oder eine vorgegebene gekrümmte Fläche im Raum überstreichen und einen gegebenen Mindestabstand zu ihr überschreiten bzw. nicht überschreiten und/oder die Änderung der Orientierung des Achsvektors zwischen den Achsen einen definierten maximalen Winkel nicht überschreitet bzw. unterschreitet oder ob bei einem bestimmten Abwinkelungszustand einer Gelenkkomponente ein Sprung des neu berechneten Drehmittelpunkts oder der neu berechneten momentanen Drehachse vorliegt, so dass auf ein Zusammenstoßen der Gelenkkomponenten geschlossen werden kann und der Ort oder Bereich des Zusammenstoßens auch lokalisiert werden kann. Ebenso kann der Computer feststellen, ob sich die momentanen Drehmittelpunkte entlang einer stetigen Geraden bewegen, oder ob die momentanen Drehachsen auf einer zum Beispiel auch gekrümmten Fläche im Raum liegen oder ob bei einem bestimmten Abwinkelungszustand einer Gelenkkomponente ein nicht stetiger Sprung des neu berechneten Drehmittelpunktes oder der neu berechneten momentanen Drehachse vorliegt, so dass auf ein Zusammenstoßen der Gelenkkomponenten geschlossen werden kann und der Ort oder Bereich des Zusammenstoßens auch lokalisiert werden kann.

Ebenso kann ergänzend oder alternativ ein oder mehrere der oben beispielhaft beschriebenen acht Kriterien verwendet werden.

Vorzugsweise ist der Computer mit einer Datenbank verbunden, in welcher zum Beispiel Informationen bezüglich der dreidimensionalen Geometrie der Gelenkkomponenten, wie zum Beispiel der Femur und/oder der Tibia oder von Gelenkimplantaten abgelegt sind. Weiterhin kann in der Datenbank auch der Verlauf einer Kurve, Fläche oder eines Volumens, zum Beispiel der Gangpolkurve relativ zur Tibia oder der Rastpolkurve zum Femur abgespeichert sein. Die Daten bezüglich der dreidimensionalen Struktur der Gelenkkomponenten oder bezüglich des Verlaufs der Drehzentren (zum Beispiel eine Gangpolkurve) können vor der Untersuchung des Bewegungsbereiches zum Beispiel mittels eines Computertomographen oder einer vorangehenden Bewegungsanalyse gewonnen oder vorgegeben werden.

Die Erfindung wird nachfolgend anhand eines Ausführungsbeispieles beschrieben. Es zeigen:
- Figuren 1A bis 1D: die Veränderung oder Verschiebung der momentanen Drehachsen eines Kniegelenkes bei einer Beugung;
- Figuren 2A und 2B: die prinzipielle Darstellung der Bewegung eines momentanen Drehmittelpunktes bei einer Roll- und einer Kipp-Bewegung;
- Figuren 3A und 3B: die Verschiebung der momentanen Drehachsen bei einem Zusammenstoß der Gelenkkomponenten;
- Figur 4: eine detaillierte Darstellung von Fig. 3A;
- Figur 5: das Verfahren zur Berechnung des momentanen Drehmittelpunktes; und
- Figur 6: Drehachsen, die definierte Flächen schneiden und Drehachsen, die sich in einem begrenzten Volumen schneiden oder sich windschief an einem Punkt am nächsten kommen.

Figur 1D zeigt ein Kniegelenk, wobei an der Tibia Ti ein Referenzstern R_{Ti} befestigt ist. Oberhalb der Tibia Ti ist der Femur Fe, welcher in zwei unterschiedlich abgewinkelten Stellungen relativ zur Tibia Ti gezeigt und mit einem Referenzstern R_{Fe} verbunden ist. Während der Bewegung des Femurs Fe, welche mittels einer Kamera C, welche mit einem Computer PC verbunden ist, der mit einer Datenbank Data verbunden ist, detektiert werden kann, wandert der momentane Drehmittelpunkt des Femurs Fe entlang der Gangpolkurve G.

Tritt ein Zusammenstoß des Femurs Fe mit der Tibia Ti auf, wie in Figur 3A und 4 gezeigt, so kommen Femur Fe und Tibia Ti bei dem Punkt P in Kontakt, so dass der neue von dem Computer PC aus den Positionsdaten der Marker R_{Fe} und R_{Ti} berechnete Drehmittelpunkt nicht mehr eine stetige Fortsetzung der Kurve G ist, sondern zu einer Stelle auf der Tibia Ti springt. Ist zum Beispiel die Abmessung des auf den Femur Fe aufgesetzten Implantates Im bekannt, so kann ein Teil der Tibia Ti von einem Chirurgen zum Beispiel mittels eines zu dem Punkt P navigierten und mit einem Referenzstern R_{I} versehenen Instrumentes I herausgeschnitten werden, wenn ein Zusammenstoßen im Bereich des Punktes P den gewünschten Bewegungsbereich des Kniegelenkes nicht ermöglicht und einschränkt.

Figur 4 zeigt die mittels des Computers PC berechneten momentanen Drehmittelpunkte COR während einer normalen Beugung oder Abwinkelung des Gelenks, welche sich in der Nähe der Gangpolkurve G bewegen, bis ein Zusammenstoßen auftritt. Während des Zusammenstoßens weichen die von dem Computer PC berechneten momentanen Drehmittelpunkte COR stark von der vorgegebenen Kurve, zum Beispiel der Gangpolkurve G, ab und sammeln sich im Bereich des Kontaktpunktes P.

Die Figuren zeigen:
- Figur 1a: Bewegung der momentanen Drehachse des Femurs über einen Beugebewe- gungsbereich
- Figur 1b: Die Drehachsen liegen in einer gekrümmten Ebene und schneiden die saggitta- le Projektionsebene
- Figur 1c: Die Schnittpunkte (Drehzentren) verlaufen entlang eines physiologisch oder kinematisch definierten Weges, z.B. der Gangpolkurve
- Figur 1d: Bewegung des Femur-Drehzentrums entlang des physiologisch oder kinema- tisch definierten Weges wie in der sagittalen Projektion gesehen
- Figur 2a: Bewegung des Drehzentrums eines Rades
- Figur 2b: Drehzentrum einer Wippe
- Figur 3a: Drehzentrum während des Zusammenstoßens (impingement)
- Figur 3b: Die Drehachsen innerhalb des Bewegungsbereiches liegen in einer gekrümm- ten Ebene. Wenn ein Zusammenstoßen auftritt, dann springen diese und kön- nen ein Linienbündel bilden, welches sich in dem Bereich des Zusammensto- ßens (Pfeil) kreuzt. In diesem Beispiel tritt der Zusammenstoß hinter der ge- zeigten Sagittalebene auf. In der sagittalen Projektion bewegen sich die Dreh- zentren weg von dem physiologisch definierten Weg und sammeln sich in der posterior distalen Ecke der sagittalen Projektionsebene
- Figur 4: Drehzentren während einer normalen Beugung bleiben nahe an einem definier- ten Weg oder Bereich bis ein Zusammenstoßen (impingement) auftritt. Wäh- rend des Zusammenstoßes weichen die Drehzentren stark von dem definierten Weg ab und sammeln sich um den Bereich des Zusammenstoßes in der sagitta- len Projektion
- Figur 5: Zwei Transformationen T1 und T2 beschreiben die Lage und Position des Fe- mur-Koordinatensystems KF vor (KF1) und nach (KF2) einer Positionsverän- derung während eines finiten Beugungsschrittes in Bezug auf das Tibia- Koordinatensystem KT. Aus diesen Matrizen können die Schraubachsenpara- meter erhalten werden: Beugungswinkel θ, Punkt s auf der Achse und Rich- tungsvektor n in Bezug auf das Tibia-Koordinatensystem KT. Die Matrizen können aus dem Tracken der Referenz-Arrays auf Femur und Tibia erhalten werden
- Figur 6: Schnittpunkte SP der Achsen mit den durch die Berandungen A und B darge- stellten Flächen (oben) und begrenztes Volumen V mit Punkten der Achsen (unten), wobei die kürzesten Abstände KA der Achsen gezeigt sind

## Patentansprüche

1. Verfahren zum Erkennen eines Zusammenstoßes von Gelenkkomponenten eines anatomischen Gelenks, wobei die räumliche Lage jeder Gelenkkomponente während einer Relativbewegung der Gelenkkomponenten detektiert wird, der momentane Drehmittelpunkt oder die momentane Drehachse aus den detektierten räumlichen Lagen der Gelenkkomponenten berechnet wird und festgestellt wird, ob sich bei einer Bewegung einer Gelenkkomponente relativ zur anderen Gelenkkomponente der berechnete momentane Drehmittelpunkt oder die berechnete momentane Drehachse in einem Erwartungsbereich bewegt oder nicht, wobei im Falle des Verlassens oder Austritts aus dem Erwartungsbereich festgestellt wird, dass ein Zusammenstoß der Gelenkkomponenten vorliegt.

2. Verfahren zum Erkennen eines Zusammenstoßes von Gelenkkomponenten eines Gelenks nach Anspruch 1, wobei festgestellt wird, ob sich bei einer Bewegung einer Gelenkkomponente relativ zur anderen Gelenkkomponente die räumlich verlaufende Drehachse mindestens mit einer vorgegebenen Strecke in einem vorgegebenen definierten Volumen, insbesondere in einem Quader, einem Zylinder oder einer Kugel befindet oder über eine definierte Fläche bewegt.

3. Verfahren zum Erkennen eines Zusammenstoßes von Gelenkkomponenten eines Gelenks nach Anspruch 1, wobei festgestellt wird, ob bei einer Bewegung einer Gelenkkomponente relativ zur anderen Gelenkkomponente die Lage oder Orientierung der ermittelten Drehachse relativ zu einer Gelenkkomponente oder einer vorherigen bestimmten Drehachse innerhalb vorgegebener Grenzen liegt.

4. Verfahren zum Erkennen eines Zusammenstoßes von Gelenkkomponenten eines Gelenks nach Anspruch 1, wobei festgestellt wird, ob bei einer Bewegung einer Gelenkkomponente relativ zur anderen Gelenkkomponente eine Konzentration von Drehachsen oder Punkten der Drehachsen innerhalb eines vorgegebenen Volumens liegt.

5. Verfahren nach einem der vorhergehenden Ansprüche, wobei zur Berechnung des momentanen Drehmittelpunktes eine Bewegungsanalyse durchgeführt wird.

6. Verfahren nach einem der vorhergehenden Ansprüche, wobei der Punkt (P) oder Bereich des Zusammenstoßes relativ zu einer Gelenkkomponente lokalisiert wird und/oder die Konzentration der Drehzentren in einem Bereich detektiert wird, um den Punkt (P) oder den Verlauf der Kipplinie zu lokalisieren.

7. Verfahren nach dem vorhergehenden Anspruch, wobei überprüft wird, ob der ermittelte Ort oder Punkt innerhalb oder außerhalb eines vordefinierten Plausibilitätsbereichs, insbesondere innerhalb einer Gelenkstruktur, wie zum Beispiel dem Femur, liegt, um falsche Orte auszuschließen, wobei der Plausibilitätsbereich eine definierte Fläche oder ein Volumen sein kann.

8. Verfahren nach einem der vorhergehenden Ansprüche, wobei ermittelt wird, ob die Drehachse oder das Drehzentrum einen vorgebenden Bereich ganz oder nur teilweise überstreicht, um zum Beispiel ein Blockieren innerhalb des Arbeitsbereiches festzustellen und von einem Kippen um einen Kollisionspunkt unterscheiden zu können.

9. Verfahren nach einem der vorhergehenden Ansprüche, wobei ein Zusammenstoß dann detektiert wird, wenn die momentanen Drehmittelpunkte um einen vorgegebenen Wert von einer vorgegebenen Kurve, insbesondere der Gangpolkurve (G), abweichen.

10. Verfahren nach einem der vorhergehenden Ansprüche, wobei dreidimensionale Modelle der Gelenkkomponenten und/oder von Implantaten verwendet werden, um eine Annäherung einer Gelenkkomponente an eine andere zu detektieren.

11. Verfahren nach einem der vorhergehenden Ansprüche, wobei ein Zusammenstoß dann detektiert wird, wenn sich die momentanen Drehmittelpunkte bei einer Bewegung des Gelenks im Wesentlichen nicht entlang einer Linie bewegen oder entlang einer Kurve bewegen und Kurvenstücke mit Lücken oder Krümmungen über vorgegebenen Grenzwerten durchlaufen werden.

12. Verfahren nach einem der vorhergehenden Ansprüche, wobei an mindestens einer Gelenkkomponente ein Referenz-Array (R_{Fe}, R_{Ti}) angebracht wird, welches zum Beispiel mit einem optischen, elektromagnetischen oder mechanischen Positionserfassungssystem oder Trackingsystem erfasst werden kann.

13. Verfahren nach einem der vorhergehenden Ansprüche, wobei ein maximaler Bewegungsbereich des Gelenkes als Bereich zwischen den Zusammenstößen der Gelenkkomponenten bei einer Bewegung von einer maximalen Gelenkauslenkung zu einer anderen maximalen Gelenkauslenkung bestimmt wird.

14. Verfahren nach einem der vorhergehenden Ansprüche, wobei ein Instrument (I) zum lokalisierten Zusammenstoßpunkt (P) navigiert wird.

15. Computerprogramm, welches, wenn es in einen Computer geladen ist oder auf einem Computer läuft, ein Verfahren nach einem der vorhergehenden Ansprüche ausführt.

16. Programmspeichermedium oder Computerprogrammprodukt mit dem Computerprogramm nach dem vorhergehenden Anspruch.

17. Vorrichtung zum Erkennen eines Zusammenstoßes von Gelenkkomponenten eines anatomischen Gelenks
mit einer Erfassungseinheit (C), welche die räumliche Position von Referenz-Arrays (RFe, RTi), welche an den Gelenkkomponenten angebracht sind, erfasst;
mit einem Computer (PC), welcher mit der Erfassungseinheit (C) verbunden ist und aus den erfassten Positionen der mit den Gelenkkomponenten verbundenen Referenz-Arrays (RFe, RTi) eine momentane Drehachse oder einen momentanen Drehmittelpunkt der relativ zueinander bewegten und durch ein Gelenk verbundenen Gelenkkomponenten berechnet, wobei der Computer (PC) weiterhin feststellt, ob sich die momentanen Drehmittelpunkte in einem Erwartungsbereich bewegen, oder ob die momentanen Drehachsen in einem Erwartungsbereich liegen, oder ob bei einem bestimmten Abwinkelungszustand einer Gelenkkomponente der Erwartungsbereich verlassen wird und zum Beispiel ein Sprung des neu berechneten Drehmittelpunktes oder der neu berechneten momentanen Drehachse vorliegt, oder ob sich bei einer Bewegung einer Gelenkkomponente relativ zur anderen Gelenkkomponente die räumlich verlaufende Drehachse mindestens mit einer vorgegebenen Strecke in einem vorgegebenen definierten Volumen befindet oder über eine definierte Fläche bewegt, oder ob bei einer Bewegung einer Gelenkkomponente relativ zur anderen Gelenkkomponente die Lage oder Orientierung der ermittelten Drehachse relativ zu einer Gelenkkomponente oder einer vorherigen bestimmten Drehachse innerhalb oder außerhalb vorgegebener Grenzen liegt, oder ob bei einer Bewegung einer Gelenkkomponente relativ zur anderen Gelenkkomponente eine Konzentration von Drehachsen innerhalb eines vorgegebenen Volumens liegt, so dass auf ein Zusammenstoßen der Gelenkkomponenten geschlossen werden kann und/oder der Ort oder Bereich des Zusammenstoßens lokalisiert werden kann.

18. Vorrichtung nach Anspruch 17 mit einer Datenbank (Data), die mit dem Computer (PC) verbunden ist und in welcher Informationen bezüglich der dreidimensionalen Geometrie der Gelenkkomponenten oder von Gelenkimplantaten und (Im) der Verlauf der Gangpolkurve (G) relativ zur Tibia abgespeichert sind.

## Claims

1. Method of detecting an impingement of joint components of an anatomical joint, whereby the spatial position of each joint component is detected during a relative movement of the joint components, the instantaneous centre of rotation or the instantaneous axis of rotation is computed from the detected spatial positions of the joint components, and as one joint component is moved relative to the other joint component, it is established whether the computed instantaneous centre of rotation or the computed instantaneous axis of rotation is moving within an anticipated range or not, and if there is a departure or variance from the anticipated range it is concluded that an impingement of the joint components exists.

2. Method of detecting an impingement of joint components of a joint as claimed in claim 1, whereby it is established whether, as one joint component is moved relative to the other joint component, the spatially extending axis of rotation is disposed at least at a given distance in a predefined given volume, in particular in a cube, a cylinder or a sphere, or is moving across a defined surface.

3. Method of detecting an impingement of joint components of a joint as claimed in claim 1, whereby it is established whether, as one joint component is moved relative to the other joint component, the position or orientation of the detected axis of rotation relative to a joint component or a previously determined axis of rotation lies within predefined limits.

4. Method of detecting an impingement of joint components of a joint as claimed in claim 1, whereby it is established whether, as one joint component is moved relative to the other joint component, a concentration of axes of rotation or points of the axes of rotation lies within a predefined volume.

5. Method as claimed in one of the preceding claims, whereby a motion analysis is run in order to compute the instantaneous centre of rotation.

6. Method as claimed in one of the preceding claims, whereby the point (P) or region of the impingement relative to one joint component is located and/or the concentration of centres of rotation in a region is detected in order to locate the point (P) or the course of the tilting line.

7. Method as claimed in the preceding claim, whereby a check is run to ascertain whether the detected site or point is inside or outside of a predefined plausibility range, in particular within a joint structure such as the femur for example, in order to rule out incorrect sites, and the plausibility range may be a defined surface or a volume.

8. Method as claimed in one of the preceding claims, whereby it is determined whether the axis of rotation or the centre of rotation overlaps a predefined range completely or only partially, for example to enable a blockage within the working range to be located and distinguished from a tilting movement about a collision point.

9. Method as claimed in one of the preceding claims, whereby an impingement is detected whenever the instantaneous centres of rotation around a predefined value deviate from a predefined curve, in particular the moving centrode (G).

10. Method as claimed in one of the preceding claims, whereby three-dimensional models of the joint components and/or implants are used to detect a movement of one joint component towards another.

11. Method as claimed in one of the preceding claims, whereby an impingement is detected whenever the instantaneous centres of rotation during a movement of the joint essentially do not move along a line or along a curve, and gaps or curvatures beyond predefined threshold values extend through parts of the curve.

12. Method as claimed in one of the preceding claims, whereby a reference array (R_{Fe}, R_{Ti}) which can be detected by means of an optical, electromagnetic or mechanical position detecting system or tracking system, for example, is applied to at least one joint component.

13. Method as claimed in one of the preceding claims, whereby a maximum range of motion of the joint is defined as being the range between the impingements of the joint components during a movement from one maximum joint deflection to another maximum joint deflection.

14. Method as claimed in one of the preceding claims, whereby an instrument (I) is navigated to the located impingement point (P).

15. Computer programme, which runs a method as claimed in one of the preceding claims when loaded onto a computer or running on a computer.

16. Programme storage medium or computer programme product with the computer programme as claimed in the preceding claim.

17. Device for detecting an impingement of joint components of an anatomical joint,
with a detection unit (C) which detects the spatial position of reference arrays (R_{Fe}, R_{Ti}) applied to the joint components;
with a computer (PC) which is connected to the detection unit (C) and computes an instantaneous axis of rotation or an instantaneous centre of rotation of the joint components moved relative to one another and connected by a joint from the detected positions of the reference arrays (R_{Fe}, R_{Ti}) connected to the joint components, and the computer (PC) also ascertains whether the instantaneous centres of rotation lie within an anticipated range, or whether the instantaneous axes of rotation lie within an anticipated range, or whether there is a variance from the anticipated range when a joint component is in a given bent state and there is a jump in the newly computed centre of rotation or the newly computed instantaneous axis of rotation, or whether the spatially extending axis of rotation is disposed at least at a predefined distance in a predefined given volume or is moving across a defined surface when one joint component is moving relative to the other joint component, or whether the position or orientation of the detected axis of rotation relative to a joint component or a previously defined axis of rotation lies inside or outside predefined limits when one joint component is moving relative to the other joint component, or whether a concentration of axes of rotation lies within a predefined volume when one joint component is moving relative to the other joint component so that it can be concluded that an impingement of the joint components exists and/or the site or region of the impingement can be located.

18. Device as claimed in claim 17, with a data bank (Data) which is connected to the computer (PC) and in which information relating to the three-dimensional geometry of the joint components or joint implants (Im) and the graph plotting the moving centrode (G) relative to the tibia are stored.

## Revendications

1. Procédé pour détecter un empiétement de composants articulaires d'une articulation anatomique, dans lequel on détecte la position spatiale de chaque composant articulaire pendant un mouvement relatif des composants articulaires, on calcule le centre de rotation instantané ou l'axe de rotation instantané à partir des positions spatiales détectées des composants articulaires et on détermine si lors d'un mouvement d'un composant articulaire par rapport à un autre composant articulaire, le centre de rotation instantané calculé ou l'axe de rotation instantané calculé s'est déplacé ou non dans une plage attendue, dans lequel on détermine en cas de sortie ou d'excursion en dehors de la plage attendue qu'il s'agit d'un empiètement des composants articulaires.

2. Procédé pour détecter un empiètement de composants articulaires d'une articulation selon la revendication 1, dans lequel on détermine si lors d'un mouvement d'un composant articulaire par rapport à un autre composant articulaire, l'axe de rotation s'étendant spatialement se trouve au moins à une distance prédéterminée dans un volume défini prédéterminé, en particulier dans un parallélépipède, un cylindre ou une sphère, ou se déplace sur une surface définie.

3. Procédé pour détecter un empiètement de composants articulaires d'une articulation selon la revendication 1, dans lequel on détermine si lors d'un mouvement d'un composant articulaire par rapport à un autre composant articulaire, la position ou l'orientation de l'axe de rotation déterminé par rapport à un composant articulaire ou à un axe de rotation déterminé précédent se situe dans des limites prédéterminées.

4. Procédé pour détecter un empiètement de composants articulaires d'une articulation selon la revendication 1, dans lequel on détermine si lors d'un mouvement d'un composant articulaire par rapport à un autre composant articulaire, une concentration d'axes de rotation ou de points des axes de rotation se situe à l'intérieur d'un volume prédéterminé.

5. Procédé selon l'une quelconque des revendications précédentes, dans lequel une analyse de mouvement est réalisée pour calculer le centre de rotation instantané.

6. Procédé selon l'une quelconque des revendications précédentes, dans lequel on localise le point (P) ou la zone de l'empiètement par rapport à un composant articulaire et/ou on détecte la concentration des centres de rotation dans une zone afin de localiser le point (P) ou l'allure de la ligne de basculement.

7. Procédé selon la revendication précédente, dans lequel on vérifie si l'emplacement ou le point déterminé se situe à l'intérieur ou à l'extérieur d'une zone de vraisemblance prédéfinie, en particulier à l'intérieur d'une structure articulaire, telle que par exemple le fémur, afin d'exclure les mauvais emplacements, dans lequel la zone de vraisemblance peut être une surface définie ou un volume.

8. Procédé selon l'une quelconque des revendications précédentes, dans lequel on détermine si l'axe de rotation ou le centre de rotation couvre une plage prédéfinie en totalité ou uniquement en partie, afin de déterminer par exemple un blocage à l'intérieur de la zone de travail et de pouvoir distinguer un basculement autour d'un point de collision.

9. Procédé selon l'une quelconque des revendications précédentes, dans lequel on peut détecter un empiètement lorsque les centres de rotation instantanés s'écartent d'une courbe prédéterminée d'une valeur prédéterminée, en particulier de la courbe roulante (G).

10. Procédé selon l'une quelconque des revendications précédentes, dans lequel on utilise des modèles tridimensionnels de composants articulaires et/ou d'implants afin de détecter un rapprochement mutuel des composants articulaires.

11. Procédé selon l'une quelconque des revendications précédentes, dans lequel on détecte un empiètement lorsque les centres de rotation instantanés ne se déplacent pas sensiblement le long d'une ligne lors d'un mouvement de l'articulation ou se déplacent le long d'une courbe, et des portions de courbe sont franchies avec des vides ou des courbures sur des valeurs limites prédéterminées.

12. Procédé selon l'une quelconque des revendications précédentes, dans lequel un réseau de référence (R_{Fe}, R_{Ti}) est appliqué à au moins un composant articulaire, lequel réseau peut être détecté par exemple avec un système de détection de position ou un système de suivi optique, électromagnétique ou mécanique.

13. Procédé selon l'une quelconque des revendications précédentes, dans lequel on détermine une plage de mouvement maximal de l'articulation sous la forme d'une zone entre les empiètements des composants articulaires lors d'un mouvement d'une excursion articulaire maximale à une autre excursion articulaire maximale.

14. Procédé selon l'une quelconque des revendications précédentes, dans lequel un instrument (I) est déplacé jusqu'au point d'empiètement (P) localisé.

15. Programme informatique qui, lorsqu'il est chargé dans un ordinateur ou exécuté sur un ordinateur, met en oeuvre un procédé selon l'une quelconque des revendications précédentes.

16. Support de mémorisation de programme ou produit de programme informatique comportant le programme informatique selon la revendication précédente.

17. Dispositif pour détecter un empiètement de composants articulaires d'une articulation anatomique comportant :
une unité de détection (C) qui détecte la position spatiale de réseaux de référence (R_{Fe}, R_{Ti}) qui sont appliqués aux composantes articulaires,
un ordinateur (PC) qui est relié à l'unité de détection (C) et qui calcule à partir des positions détectées des réseaux de référence (R_{Fe}, R_{Ti}) associés aux composants articulaires, un axe de rotation instantané ou un centre de rotation instantané des composants articulaires mobiles l'un par rapport à l'autre et reliés par une articulation, dans lequel l'ordinateur (PC) détermine en outre si les centres de rotation instantanés se déplacent sur une plage attendue, ou si les axes de rotation instantanés sont dans une plage attendue, ou si une sortie de la plage attendue se produit lors d'un état déterminé de déviation d'un composant articulaire et s'il s'agit, par exemple, d'un saut du nouveau centre de rotation calculé ou du nouvel axe de rotation instantané calculé, ou si lors d'un mouvement d'un composant articulaire par rapport à un autre composant articulaire, l'axe de rotation s'étendant spatialement se trouve à une distance prédéterminée au moins dans un volume défini prédéterminé ou se déplace sur une surface définie, ou si lors d'un mouvement d'un composant articulaire par rapport à un autre composant articulaire, la position ou l'orientation de l'axe de rotation déterminé par rapport à un composant articulaire ou à un axe de rotation déterminé précédent se situe à l'intérieur ou à l'extérieur de limites prédéfinies, ou si lors d'un mouvement d'un composant articulation par rapport à l'autre composant articulaire, une concentration d'axes de rotation a lieu à l'intérieur d'un volume prédéfini, de telle sorte qu'on peut conclure à un empiètement de composants articulaires et/ou on peut localiser l'emplacement ou la zone de l'empiètement.

18. Dispositif selon la revendication 17 comportant une base de données (Données) qui est reliée à un ordinateur (PC) et dans lequel sont mémorisées des informations concernant la géométrie tridimensionnelle des composants articulaires ou d'implants articulaires (Im) et l'allure de la courbe roulante (G) par rapport au tibia.
